# EUROPEAN PATENT APPLICATION

(11) **EP 4 321 161 A1**
(43) Date of publication of application: **14.02.2024**
(21) Application number: 22784860.3
(22) Date of filing: 31.03.2022
(51) Int. Cl.: A61K 31/4375, A61K 31/352, A61P 13/12, A23L 33/10

(54) **COMPOUND FOR PREVENTION OR TREATMENT OF KIDNEY DISEASE**

(30) Priority: 06.04.2021 KR 20210044583
(71) Applicant: Oncocross Co., Ltd., Seoul 04168 (KR)
(72) Inventor: KIM, Yi Rang, Sejong 30064 (KR)
(74) Representative: Isarpatent
(86) International application number: PCT/KR2022/004621
(87) International publication number: WO 2022/215950

(57) **Abstract**

The present disclosure relates to a use of (R)-4-{(R)-1-[7-(3,4,5-trimethoxyphenyl)-[1,6] naphthyridin-5-yloxy]-ethyl}pyrrolidin-2-one in treating kidney disease. According to the present disclosure, (R)-4-{(R)-1-[7-(3,4,5-trimethoxy-phenyl)-[1,6] naphthyridin-5-yloxy]-ethyl}pyrrolidin-2-one of the present disclosure palliates clinical symptoms and alleviates renal failure and renal injury through reduction of blood glucose levels and insulin resistance in diabetic nephrosis animal models. Thus, the compound may be advantageously applied to a use for preventing or treating kidney diseases, especially diabetic renal diseases.

## Description

### [Technical Field]

The present disclosure relates to a use of (R)-4-{(R)-1-[7-(3,4,5-trimethoxyphenyl)-[1,6] naphthyridin-5-yloxy]-ethyl}pyrrolidin-2-one in treating kidney disease.

### [Background Art]

Diabetes mellitus is a metabolic disease characterized by hyperglycemia caused by defects in insulin secretion or insulin action, and a chronic degenerative disease that occurs due to a lack of insulin or a decrease in a biological effect of insulin in target cells and persists for a long time, resulting in complications such as renal failure. The diabetes mellitus causes various complications from the early stages, and is accompanied by acute complications such as hypoglycemia, ketoacidosis, diabetic coma, local and systemic bacterial infection, and chronic complications such as diabetic nephropathy, hypertension, diabetic retinopathy, cataracts, neuropathy, and cardiovascular complications. The diabetes mellitus is the third most serious disease worldwide and the fourth leading cause of death in Korea, and shortens the lifespan by 5 to 10 years due to various complications. The diabetes mellitus is a metabolic disorder syndrome characterized by hyperglycemia caused by defects in insulin action, insulin secretion, or both, and as hyperglycemia continues chronically, the hyperglycemia causes disorders in not only carbohydrate metabolism but also lipid and protein metabolisms so that complications in the retina, kidneys, nerves, cardiovascular system, etc., are caused resulting in fatal problems such as pain and shortened lifespan for patients. Recently, the number of patients with diabetes mellitus is increasing, while the importance of developing therapeutic agents for diabetes mellitus is emphasized in situations where continuous and appropriate treatment is difficult.

Currently, the treatment of diabetes mellitus is broadly divided into diet therapy, exercise therapy, and drug therapy and is determined depending on a patient's condition. Since the diabetes mellitus is almost impossible to completely cure diabetes after the onset of diabetes, the treatment is aimed at secondary prevention of palliating symptoms and preventing acute and chronic complications. In other words, in the diabetes mellitus, prevention and treatment of complications are more important than the disease itself, and thus, the purpose of management is always to maintain blood glucose levels and prevent complications. Currently, therapeutic agents for diabetes mellitus include oral hypoglycemic agents such as sulfonylureas, biguanides, alpha-glucosidase inhibitors, and thiazolidinediones, and insulin therapy, pancreas transplantation, oriental therapy, and alternative medicine therapy have been used. Since the diabetes mellitus is caused by various pathogenic mechanisms, the treatments are also diverse. Furthermore, in many cases, existing classical treatments alone do not provide satisfactory results, so that new treatments are required. Currently, many studies have been conducted on some insulin stimulants, insulin action enhancers, drugs that overcome insulin resistance, drugs that have an insulin-like effect on target tissues, gluconeogenesis inhibitors, insulin antagonist hormone inhibitors, drugs for delaying carbohydrate absorption, and amylin analogues, but many thereof are still at an inadequate stage for use in the human body, or side effects due to toxicity have been a problem.

Diabetic renal disease (diabetic nephropathy) is a complication caused by diabetes mellitus, and is a disease in which the kidney function deteriorates due to continuous damage to the glomeruli inside the kidney by hypertension and the like. The diabetic renal disease is the most common cause of end-stage renal failure and is known to occur in 20 to 40% of patients with diabetes mellitus. Such a diabetic renal disease is the most important cause of chronic renal failure and account for a large proportion of all dialysis patients. The diabetic renal disease usually has no symptoms in the early stages, and as the diabetic renal disease progresses, there are no obvious symptoms other than proteinuria in a urine test. Accordingly, the onset of diabetic renal disease is diagnosed by the finding of microalbuminuria (urinary albumin secretion of 30 to 299 mg/24h). However, as described above, since a time point at which microalbuminuria symptoms are found is also after diabetic renal diseases have progressed significantly, it is not easy to diagnose diabetic renal diseases early, and the diabetic renal diseases are most often found after the kidney diseases have already progressed considerably. In addition, kidney diseases, including renal failure, caused by microvascular disorders, not only have high incidence, but also are not very effective in any attempt to prevent the progression of the diseases when once occurring. In the case of pathogenic kidney disease, the abnormal proliferation of renal mesangial cells leads to hypertrophy of the mesangial tissue to eventually thicken the basement membranes of glomeruli and tubules and narrow the glomerular vascular diameter. As symptoms of increase of glomerular mesangium, accumulation of extracellular lipids, glomerulosclerosis, and tubulointerstitial fibrosis progress, kidney function gradually weakens, ultimately leading to chronic renal failure. Another clinical significance of diabetic renal diseases is a significant increase in mortality. Currently, it has been reported that approximately 40 to 50% of diabetic patients die from renal failure, and the mortality rate of patients with type 1 diabetes mellitus accompanied by overt proteinuria to normal people is significantly higher than that of patients with type 1 diabetes mellitus with normal albuminuria and normal people. That is, even among patients with diabetes mellitus, the mortality rate significantly increases in patients with kidney diseases caused by diabetes mellitus. The proportion of diabetic renal diseases is gradually increasing in kidney diseases along with an increase in the number of patients with diabetes mellitus, and in particular, the number of patients with kidney diseases caused by Type 2 diabetes mellitus is continuously increasing. However, to date, the cause of diabetic nephropathy has not yet been accurately identified, and it is considered that complex factors such as hemodynamic changes in the kidney, glucosylation of glomerular proteins, and accumulation of sorbitol are involved. Accordingly, there are few drugs with effective preventive or therapeutic effects on diabetic renal diseases, and drugs that have been conventionally used may cause many side effects due to long-term use, such as hyperkalemia and acute renal failure due to even functional renal artery dysfunction. Accordingly, the development of new drugs for safe and effective prevention and treatment of diabetic renal diseases is very important.

Meanwhile, chronic kidney disease (CKD) refers to a disease in which kidney function is gradually and progressively lost due to various causes. According to the commonly used definition of the National Kidney Foundation, chronic kidney diseases are defined depending on the presence or absence of renal injury and the level of kidney function, that is, defined as that there is evidence of renal injury such as proteinuria or hematuria, or a state where the glomerular filtration rate (GFR) indicating renal function is reduced to less than 60 ml/min/1.73 m² has chronically continued for 3 months or more.

### [Disclosure]

### [Technical Problem]

An object of the present disclosure is to provide a pharmaceutical composition for preventing or treating kidney diseases.

Another object of the present disclosure is to provide a food composition for preventing or palliating kidney diseases.

Yet another object of the present disclosure is to provide a method for treating kidney diseases.

Yet another object of the present disclosure is to provide a use for use in the preparation of a pharmaceutical composition for preventing or treating kidney diseases.

### [Technical Solution]

One aspect of the present disclosure provides a pharmaceutical composition for preventing or treating kidney diseases containing (R)-4-{(R)-1-[7-(3,4,5-trimethoxy-phenyl)-[1,6] naphthyridin-5-yloxy]-ethyl}pyrrolidin-2-one or a pharmaceutically acceptable salt thereof as an active ingredient.

Another aspect of the present disclosure provides a food composition for preventing or palliating kidney diseases containing (R)-4-{(R)-1-[7-(3,4,5-trimethoxy-phenyl)-[1,6] naphthyridin-5-yloxy]-ethyl}pyrrolidin-2-one or a pharmaceutically acceptable salt thereof as an active ingredient.

Yet another aspect of the present disclosure provides a method for treating kidney diseases including administering (R)-4-{(R)-1-[7-(3,4,5-trimethoxy-phenyl)-[1,6] naphthyridin-5-yloxy]-ethyl}pyrrolidin-2-one or a pharmaceutically acceptable salt thereof in a pharmaceutically effective dose to a subject with kidney diseases.

Yet another aspect of the present disclosure provides a use of (R)-4-{ (R)-1-[7-(3,4,5-trimethoxy-phenyl)-[1,6] naphthyridin-5-yloxy]-ethyl}pyrrolidin-2-one or a pharmaceutically acceptable salt thereof for use in the preparation of a pharmaceutical composition for the prevention and treatment of kidney diseases.

Yet another aspect of the present disclosure provides a use of (R)-4-{ (R)-1-[7-(3,4,5-trimethoxy-phenyl)-[1,6] naphthyridin-5-yloxy]-ethyl }pyrrolidin-2-one and cromolyn or pharmaceutically acceptable salts thereof for use in the preparation of a pharmaceutical composition for the prevention and treatment of kidney diseases.

### [Advantageous Effects]

According to the present disclosure, (R)-4-{(R)-1-[7-(3,4,5-trimethoxyphenyl)-[1,6] naphthyridin-5-yloxy]-ethyl}pyrrolidin-2-one of the present disclosure palliates clinical symptoms and alleviates renal failure and renal injury through reduction of blood glucose levels and insulin resistance in diabetic nephrosis animal models. Thus, the compound can be advantageously applied to a use for preventing or treating kidney diseases, especially diabetic renal diseases.

### [Description of Drawings]

FIG. 1 is a diagram confirming histopathological changes in liver, kidney, and pancreas of a normal control group and a negative control group.
FIG. 2 is a diagram confirming histopathological changes in liver and kidney according to a dose of (R)-4-{(R)-1-[7-(3,4,5-trimethoxy-phenyl)-[1,6] naphthyridin-5-yloxy] -ethyl }pyrrolidin-2-one (BI-1002494).

### [Best Mode for the Invention]

Hereinafter, an embodiment of the present disclosure will be described in detail with reference to the accompanying drawings. However, the following embodiments are presented as examples for the present disclosure, and when it is determined that a detailed description of well-known technologies or configurations known to those skilled in the art may unnecessarily obscure the gist of the present disclosure, the detailed description thereof may be omitted, and the present disclosure is not limited thereto. Various modifications and applications of the present disclosure are possible within the description of claims to be described below and the equivalent scope interpreted therefrom.

Terminologies used herein are terminologies used to properly express embodiments of the present disclosure, which may vary according to a user, an operator's intention, or customs in the art to which the present disclosure pertains. Therefore, these terminologies used herein will be defined based on the contents throughout the specification. Throughout the specification, unless explicitly described to the contrary, when a certain part "comprises" a certain component, it will be understood to imply the inclusion of stated elements but not the exclusion of any other elements.

All technical terms used in the present disclosure, unless otherwise defined, have the meaning as commonly understood by those skilled in the related art of the present disclosure. In addition, although preferred methods and samples are described herein, similar or equivalent methods and samples thereto are also included in the scope of the present disclosure. The contents of all publications disclosed as references in this specification are incorporated in the present disclosure.

In one aspect, the present disclosure relates to a pharmaceutical composition for preventing or treating kidney diseases containing (R)-4-{(R)-1-[7-(3,4,5-trimethoxy-phenyl)-[1,6] naphthyridin-5-yloxy]-ethyl}pyrrolidin-2-one or a pharmaceutically acceptable salt thereof as an active ingredient.

In one embodiment, the (R)-4-{(R)-1-[7-(3,4,5-trimethoxy-phenyl)-[1,6] naphthyridin-5-yloxy]-ethyl}pyrrolidin-2-one may be a compound represented by Chemical Formula 1 below.

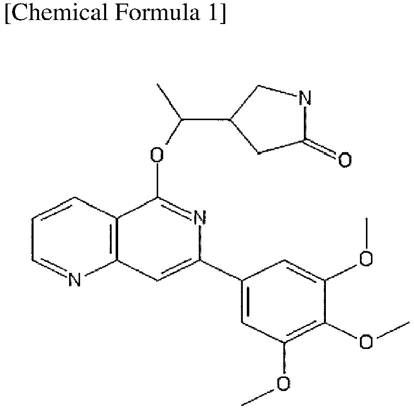

In one embodiment, the kidney diseases may be one or more selected from the group consisting of acute kidney injury, end-stage kidney disease (ESKD), renal failure, systemic lupus erythematosus, diabetic renal disease (DN), IgA nephritis (IgAN), HIV-related nephropathy, chronic kidney disease, focal segmental glomerulosclerosis (FSGS), minimal change nephrotic syndrome (MCD), and xanthine oxidase deficiency, more desirably diabetic renal disease (or diabetic nephropathy), and most desirably Type 2 diabetic nephropathy caused by insulin resistance.

In one embodiment, the composition of the present disclosure may inhibit apoptosis of the renal medullar.

In one embodiment, the composition may inhibit the calcification of the renal medullar.

In one embodiment, the composition of the present disclosure may further include cromolyn sodium (OC-101).

In one embodiment, the cromolyn may be a compound represented by Chemical Formula 2 below:

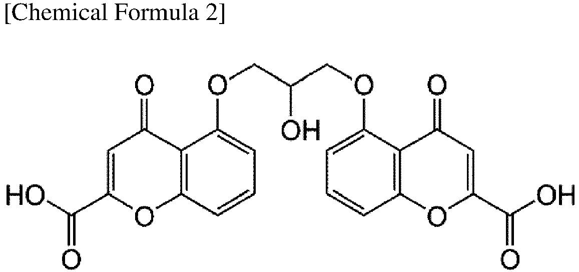

In one embodiment, a pharmaceutically acceptable salt of the cromolyn may be selected from the group consisting of alkali metal salts, alkaline earth metal salts, salts with inorganic acids, salts with organic acids, and salts with acidic amino acids, and most desirably cromolyn sodium.

In one embodiment, the (R)-4-{(R)-1-[7-(3,4,5-trimethoxy-phenyl)-[1,6] naphthyridin-5-yloxy]-ethyl}pyrrolidin-2-one and the cromolyn may be administered simultaneously, separately, or sequentially.

In one embodiment, the (R)-4-{(R)-1-[7-(3,4,5-trimethoxy-phenyl)-[1,6] naphthyridin-5-yloxy]-ethyl}pyrrolidin-2-one and the cromolyn may be each contained in the composition at concentrations of 5 to 100 mg/kg, respectively.

The present disclosure includes not only a compound represented by Chemical Formula 1 or 2, but also pharmaceutically acceptable salts thereof, and all solvates, hydrates, racemates or stereoisomers, which are able to be prepared therefrom.

The (R)-4-{(R)-1-[7-(3,4,5-trimethoxy-phenyl)-[1,6] naphthyridin-5-yloxy]-ethyl}pyrrolidin-2-one represented by Chemical Formula 1 or 2 of the present disclosure and the cromolyn may each be used in the form of pharmaceutically acceptable salts. As the salts, acid addition salts formed with pharmaceutically acceptable free acids are useful. The acid addition salts are obtained from inorganic acids such as hydrochloric acid, nitric acid, phosphoric acid, sulfuric acid, hydrobromic acid, hydroiodic acid, nitrous acid or phosphorous acid, and non-toxic organic acids such as aliphatic mono- and di-carboxylates, phenyl-substituted alkanoates, hydroxy alkanoates and alkanedioates, aromatic acids, aliphatic and aromatic sulfonic acids. These pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, hydroxybutyrate, glycolate, malate, tartrate, methanesulfonate, propanesulfonate, naphthalene-1-sulfonate, naphthalene-2-sulfonate or mandelate.

The acid addition salts according to the present disclosure may be prepared by a conventional method, for example, by dissolving the compound in an excess acidic aqueous solution and precipitating the salt using a water-miscible organic solvent, such as methanol, ethanol, acetone or acetonitrile. In addition, the acid addition salts may also be prepared by evaporating and drying a solvent or excess acid from the mixture or by suction-filtering the precipitated salt. In addition, pharmaceutically acceptable metal salts may be prepared using bases. Alkali metal salts or alkaline earth metal salts may be obtained, for example, by dissolving the compound in an excess alkali metal hydroxide or alkaline earth metal hydroxide solution, filtering a non-dissolved compound salt, and then evaporating and drying a filtrate. At this time, as the metal salt, it is pharmaceutically suitable to prepare a sodium, potassium or calcium salt. Further, silver salts corresponding thereto may be obtained by reacting the alkali metal or alkaline earth metal salt with a suitable silver salt (e.g., silver nitrate).

The pharmaceutical composition of the present disclosure may further include known therapeutic agents for kidney disease in addition to the (R)-4-{(R)-1-[7-(3,4,5-trimethoxy-phenyl)-[1,6] naphthyridin-5-yloxy]-ethyl}pyrrolidin-2-one and the cromolyn, or salts thereof, and may be combined with other known treatments for the treatment of these diseases.

As used herein, the term "prevention" means all actions that inhibit or delay the occurrence, spread, and recurrence of metabolic diseases by administration of the pharmaceutical composition according to the present disclosure. The term "treatment" means all actions that improve or beneficially change the symptoms of metabolic diseases by administering the composition of the present disclosure. Those skilled in the art to which the present disclosure pertains will be able to determine the degree of palliation, enhancement and treatment by knowing the exact criteria of a disease for which the composition of the present disclosure is effective by referring to data presented by the Korean Academy of Medical Sciences, etc.

As used herein, the term "therapeutically effective dose" used in combination with the active ingredients means an amount effective to prevent or treat a target disease, and the therapeutically effective dose of the composition of the present disclosure may vary depending on several factors, such as a method of administration, a target site, and the condition of a patient. Accordingly, when used in the human body, the dose should be determined as an appropriate amount in consideration of both safety and efficiency. It is also possible to estimate the amount used in humans from the effective dose determined through animal experiments. These matters to be considered when determining the effective dose are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present disclosure is administered in a pharmaceutically effective dose. As used herein, the term "pharmaceutically effective dose" refers to an amount enough to treat diseases at a reasonable benefit/risk ratio applicable to medical treatment and enough not to cause side effects. The effective dose level may be determined according to factors including a health condition of a patient, a type of kidney disease, an occurrence cause of kidney disease, severity, drug activity, sensitivity to a drug, an administration method, an administration time, an administration route and excretion rate, a treatment period, and drugs used in combination or concurrently, and other factors well-known in medical fields. The composition of the present disclosure may be administered as an individual therapeutic agent or in combination with other therapeutic agents, and may be administered sequentially or simultaneously with existing therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present disclosure may include carriers, diluents, excipients, or a combination of two or more thereof, which are commonly used in biological agents. As used herein, the term "pharmaceutically acceptable" means that the composition exhibits non-toxic properties to cells or humans exposed to the composition. The carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared desirably according to each disease or ingredient using as a suitable method in the art or a method disclosed in Remington's Pharmaceutical Science (Mack Publishing Company, Easton PA, 18th, 1990).

In one embodiment, the pharmaceutical composition may be one or more formulations selected from the group consisting of oral formulations, external preparations, suppositories, sterile injections and sprays, and more desirably oral or injective formulations.

As used herein, the term "administration" means providing a predetermined substance to a subject or patient by any suitable method, and parenteral administration (e.g., applied in an injection formulation intravenously, subcutaneously, intraperitoneally or topically) or oral administration may be performed according to a desired method. The dose range may vary depending on the body weight, age, sex, and health condition of a patient, a diet, an administration time, an administration method, an excretion rate, and the severity of a disease. Liquid formulations for oral administration of the composition of the present disclosure correspond to suspensions, internal solutions, emulsions, syrups, etc., and may include various excipients, such as wetting agents, sweeteners, fragrances, and preservatives in addition to water and liquid paraffin, which are commonly used simple diluents. Formulations for parenteral administration include sterilized aqueous solutions, non-aqueous solvents, suspensions, emulsions, lyophilized agents, and suppositories. The pharmaceutical composition of the present disclosure may also be administered by any device capable of transferring an active substance to a target cell. Desired methods of administration and formulations are intravenous injections, subcutaneous injections, intradermal injections, intramuscular injections, or drop injections. The injections may be prepared by using aqueous solvents such as a physiological saline solution and a ringer solution, and non-aqueous solvents such as vegetable oils, higher fatty acid esters (e.g., ethyl oleate), and alcohols (e.g., ethanol, benzyl alcohol, propylene glycol, or glycerin). The injections may include pharmaceutical carriers, such as a stabilizer for the suppression of degeneration (e.g., ascorbic acid, sodium hydrogen sulfite, sodium pyrosulfite, BHA, tocopherol, and EDTA), an emulsifier, a buffer for pH control, and a preservative to inhibit microbial growth (e.g., phenyl mercury nitrate, thimerosal, benzalkonium chloride, phenol, cresol, and benzyl alcohol).

As used herein, the term "subject" refers to all animals including monkeys, cows, horses, sheep, pigs, chickens, turkeys, quails, cats, dogs, mice, rats, rabbits or guinea pigs including humans who have developed or may develop the kidney diseases, and the pharmaceutical composition of the present disclosure may be administered to a subject to effectively prevent or treat the diseases. The pharmaceutical composition of the present disclosure may be administered in combination with existing therapeutic agents.

The pharmaceutical composition of the present disclosure may further include pharmaceutically acceptable additives. At this time, the pharmaceutically acceptable additives may use starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, white sugar, dextrose, sorbitol, talc and the like. The pharmaceutically acceptable additive according to the present disclosure is desirably included in an amount of 0.1 part by weight to 90 parts by weight based on the composition, but is not limited thereto.

In one aspect, the present disclosure relates to a food composition for preventing or palliating kidney diseases containing (R)-4-{(R)-1-[7-(3,4,5-trimethoxy-phenyl)-[1,6] naphthyridin-5-yloxy]-ethyl}pyrrolidin-2-one or a pharmaceutically acceptable salt thereof as an active ingredient.

In one embodiment, the kidney diseases may be diabetic renal diseases.

In one embodiment, the kidney diseases may be one or more selected from the group consisting of acute kidney injury, end-stage kidney disease (ESKD), renal failure, systemic lupus erythematosus, diabetic renal disease (DN), IgA nephritis (IgAN), HIV-related nephropathy, chronic kidney disease, focal segmental glomerulosclerosis (FSGS), minimal change nephrotic syndrome (MCD), and xanthine oxidase deficiency, more desirably diabetic renal disease (or diabetic nephropathy), and most desirably Type 2 diabetic nephropathy caused by insulin resistance.

In one embodiment, the composition of the present disclosure may inhibit apoptosis of the renal medullar.

In one embodiment, the composition may inhibit the calcification of the renal medullar.

In one embodiment, the composition of the present disclosure may further include cromolyn.

When the composition of the present disclosure is used as the food composition, the composition may be added as it is or used with other foods or food ingredients, and may be appropriately used according to a general method. The composition may include food acceptable supplement additives in addition to the active ingredients, and the mixing amount of the active ingredients may be appropriately determined depending on the purpose of use (prevention, health or therapeutic treatment).

As used herein, the term "food supplement additive" means a component that may be supplementally added to food, and may be appropriately selected and used by those skilled in the art as being added to prepare a health functional food of each formulation. Examples of the food supplement additives include various nutrients, vitamins, minerals (electrolytes), flavors such as synthetic and natural flavors, colorants and fillers, pectic acid and salts thereof, alginic acid and salts thereof, organic acids, protective colloidal thickeners, pH adjusters, stabilizers, preservatives, glycerin, alcohols, and carbonation agents used in carbonated drinks, but the types of food supplement additives of the present disclosure are not limited by the examples.

The food composition of the present disclosure may include a health functional food. As used herein, the term "health functional food" refers to food prepared and processed in the form of tablets, capsules, powders, granules, liquids and pills using raw materials or ingredients having functionalities useful to the human body. Here, the 'functionality' means regulating nutrients to the structure and function of the human body or obtaining effects useful for health applications such as physiological action. The health functional food of the present disclosure is able to be prepared by methods to be commonly used in the art and may be prepared by adding raw materials and ingredients which are commonly added in the art in preparation. In addition, the formulations of the health functional food may also be prepared with any formulation recognized as a health functional food without limitation. The food composition of the present disclosure may be prepared in various types of formulations, and unlike general drugs, the food composition has an advantage that there is no side effect that may occur when taking a long-term use of the drug using the food as a raw material, and has excellent portability, but the health functional food of the present disclosure may be taken as supplements to enhance the effects of anticancer drugs.

In addition, there is no limitation in a type of health food in which the composition of the present disclosure may be used. In addition, the composition including the compound of the present disclosure as the active ingredient may be prepared by mixing known additives with other suitable auxiliary ingredients that may be contained in the health functional food according to the selection of those skilled in the art. Examples of foods to be added include meat, sausage, bread, chocolate, candy, snacks, confectionery, pizza, ramen, other noodles, gum, dairy products including ice cream, various soups, beverages, tea, drinks, alcoholic beverages, and vitamin complexes, and may be prepared to be added to extract, tea, jelly, and juice prepared by using the compound according to the present disclosure as a main ingredient.

In an aspect, the present disclosure relates to a method for treating kidney diseases including administering (R)-4-{(R)-1-[7-(3,4,5-trimethoxy-phenyl)-[1,6] naphthyridin-5-yloxy]-ethyl}pyrrolidin-2-one or a pharmaceutically acceptable salt thereof in a pharmaceutically effective dose to a subject with kidney diseases.

In one embodiment, the method may further include administering cromolyn or a pharmaceutically acceptable salt thereof in a pharmaceutically effective amount to a subject with kidney diseases.

In an aspect, the present disclosure relates to a use of (R)-4-{(R)-1-[7-(3,4,5-trimethoxy-phenyl)-[1,6] naphthyridin-5-yloxy]-ethyl}pyrrolidin-2-one or a pharmaceutically acceptable salt thereof for use in the preparation of a pharmaceutical composition for the prevention and treatment of kidney diseases.

In an aspect, the present disclosure relates to a use of (R)-4-{(R)-1-[7-(3,4,5-trimethoxy-phenyl)-[1,6] naphthyridin-5-yloxy]-ethyl}pyrrolidin-2-one and cromolyn or pharmaceutically acceptable salts thereof for use in the preparation of a pharmaceutical composition for the prevention and treatment of kidney diseases.

### [Modes for the Invention]

Hereinafter, the present disclosure will be described in more detail with reference to the following Examples. However, the following Examples are only intended to embody the contents of the present disclosure, and the present disclosure is not limited thereto.

### Example 1. Construction of diabetic nephrosis animal models and administration of substances

STZ (Sigma-aldrich, S0130, 40 mg/kg b.w., in 0.1 M citrate buffer pH 4.5) was intraperitoneally administered to 9-week-old male mice (C57BL/6) once/1 day for 5 days to perform a first disease induction. After 7 weeks of the first induction, a second induction was performed using the same method as the first induction. For 5 days of an STZ administration period for the first induction, 10% sucrose was administered as drinking water. In addition, after 14 days of the second administration, blood glucose levels were confirmed to confirm the induction rate, and the induced animals were separated into groups. Experimental substances (R)-4-{(R)-1-[7-(3,4,5-trimethoxy-phenyl)-[1,6] naphthyridin-5-yloxy]-ethyl}pyrrolidin-2-one (BI-1002494) and cromolyn (OC-101) were administered orally into the stomach at 10 mL/kg using an administration syringe (sonde) before 16:00 daily for 12 weeks at a frequency of 7 days/week at doses shown in Table 1 below, and dexamethasone was orally administered as a positive control substance.

**[Table 1]**

| Group | | Administering substance | Dose (mg/kg b.w.) | Dosage amount (mL/kg) | Sex | Animal number |
|---|---|---|---|---|---|---|
| G1 | Normal control | Sterile distilled water | - | 10 | Male | 10 |
| G2 | Negative control | Disease-inducing + Sterile distilled water | - | 10 | Male | 10 |
| G3 | Positive control | Disease-inducing + Dexamethasone | 2 | 10 | Male | 10 |
| G4 | Test substance administer ed group | Disease-inducing + BI-1002494 | 12.5 | 10 | Male | 10 |
| G5 | | Disease-inducing + BI-1002494 | 25 | 10 | Male | 10 |
| G6 | | Disease-inducing + BI-1002494 | 50 | 10 | Male | 10 |
| G7 | | Disease-inducing + OC-101 | 50 | 10 | Male | 10 |

### Example 2. Confirmation of clinical symptoms

### 2-1. Confirmation of body weights

As a result of confirming the body weight of mice in each group of Example 1, the body weight of a negative control group was statistically significantly reduced after week 7 of administration compared to that of a normal control group, and no statistically significant difference was observed in a positive control group. In test substance administered groups, as compared to the negative control group, in a 12.5 mg/kg administered group of (R)-4-{(R)-1-[7-(3,4,5-trimethoxy-phenyl)-[1,6] naphthyridin-5-yloxy]-ethyl}pyrrolidin-2-one, after week 5 of administration, the body weight was statistically significantly increased, and in 25 mg/kg and 50 mg/kg administered groups, after week 8, the body weights were statistically significantly increased (Table 2).

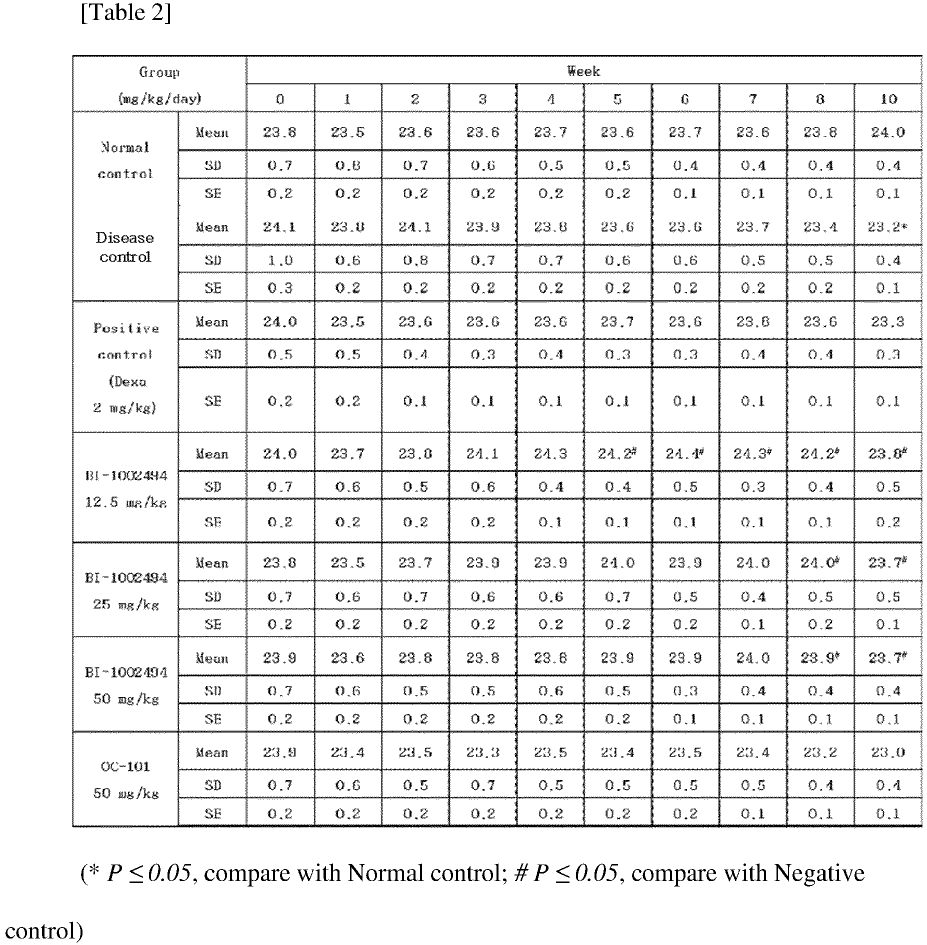

### 2-2. Confirmation of feed intake

As a result of confirming the feed intake of mice in each group of Example 1, the feed intake of a negative control group was statistically significantly increased after weeks 2, 3, 4, 6 and 8 of administration compared to that of a normal control group, and no statistically significant difference was observed in a positive control group compared to the normal control group. In test substance administered groups, as compared to the negative control group, in a 12.5 mg/kg administered group of (R)-4-{(R)-1-[7-(3,4,5-trimethoxy-phenyl)-[1,6] naphthyridin-5-yloxy]-ethyl}pyrrolidin-2-one, after week 4 of administration, in a 25 mg/kg administered group, after week 6 of administration, and in a 50 mg/kg administered group, after week 8 of administration, the feed intakes were statistically significantly decreased (Table 3).

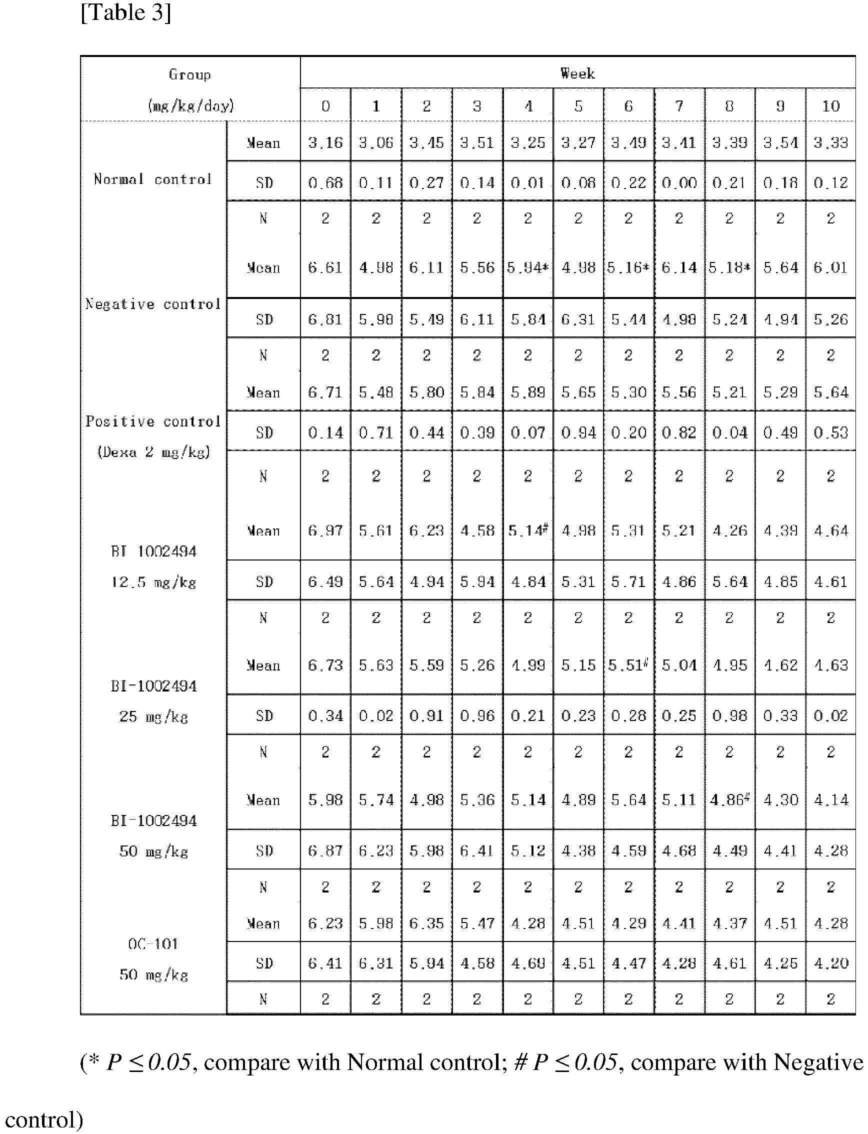

### 2-3. Confirmation of drinking water intake

As a result of confirming the drinking water intake of mice in each group of Example 1, the drinking water intake in the negative control group was statistically significantly increased at weeks 0 to 4, 6, and 7 of administration compared to that of the normal control group (Table 4).

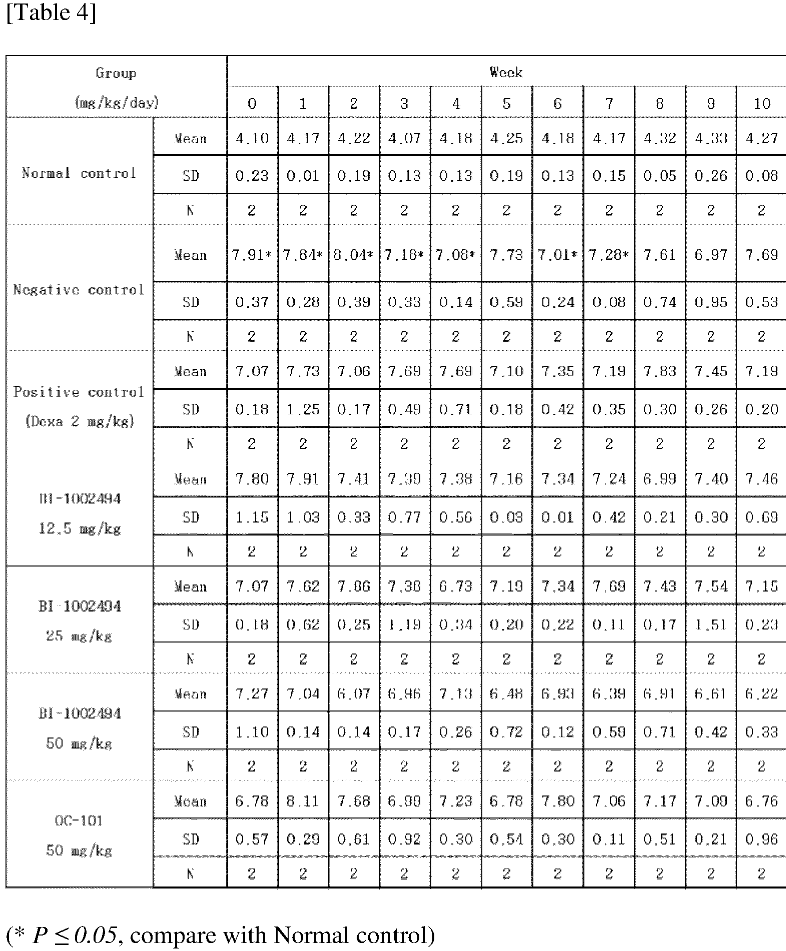

### Example 3. Confirmation of changes in blood glucose levels

Before disease induction, excluding week 9 of administration of experimental substances to mice in each group of Example 1 above, after 2 weeks after the first induction (based on a first administration date), and after 6 weeks and 2 weeks of the second induction (based on the first administration date), blood was collected from the tail vein and blood glucose levels were measured using a blood glucose level meter (Accu-Check, Roche), and fasting was performed for 4 hours from 9:30 a.m. to 1:30.

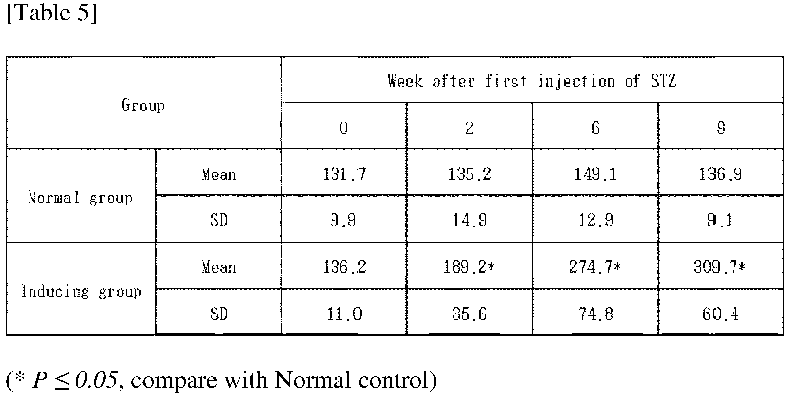

As a result, compared to the control group, a statistically significant increase in blood glucose levels was observed in a disease-inducing group from 2 weeks after the first STZ administration, and in such a change, a difference was increased even at week 6 and week 9 of administration (Table 5).

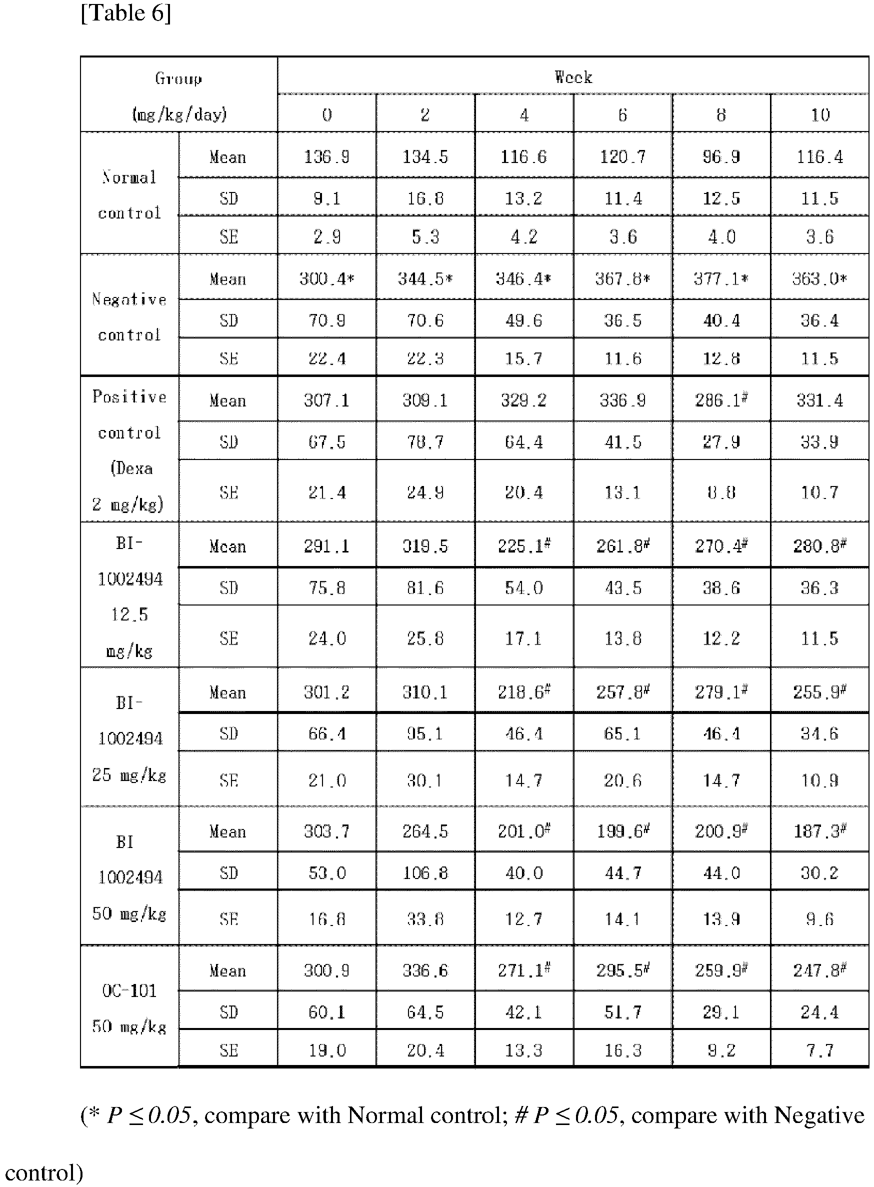

In addition, during the period before administration of the experimental substances, in the negative control group, blood glucose levels were increased statistically significantly compared to those of the normal control group, and at week 8 of administration, the blood glucose levels were decreased statistically significantly, and in the administered group of (R)-4-{(R)-1-[7-(3,4,5-trimethoxy-phenyl)-[1,6] naphthyridin-5-yloxy]-ethyl}pyrrolidin-2-one, the blood glucose levels were decreased significantly compared to those of the positive control group (Table 6).

### Example 4. Confirmation of blood biochemical change

After the observation period for mice of each group in Example 1 was terminated, all surviving animals were fasted in the same manner as when measuring blood glucose levels, and then anesthetized by inhaling isoflurane, and blood was collected from the abdominal vena cava through laparotomy. After the blood collection was completed, the veins and arteries were cut under anesthesia and exsanguinated. The collected blood was left at room temperature for 30 minutes or more and then centrifuged at 3,000 rpm for 20 minutes. Thereafter, glucose, Blood urea nitrogen (BUN), and creatinine were analyzed using an automated blood biochemistry analyzer (Hitachi 3100, Hitachi Co. Ltd., Japan). Indoxyl sulfate (a substance that was mainly produced in the intestines to cause renal injury, and a substance that caused end-stage renal failure by advancing renal function damage in patients with renal failure during hemodialysis) was analyzed using LC MS/MS. In addition, blood insulin was analyzed according to a method represented by a commercial Mouse Insulin ELISA KIT (#AKRIN-011T, Sibayagi Co. Ltd., Japan).

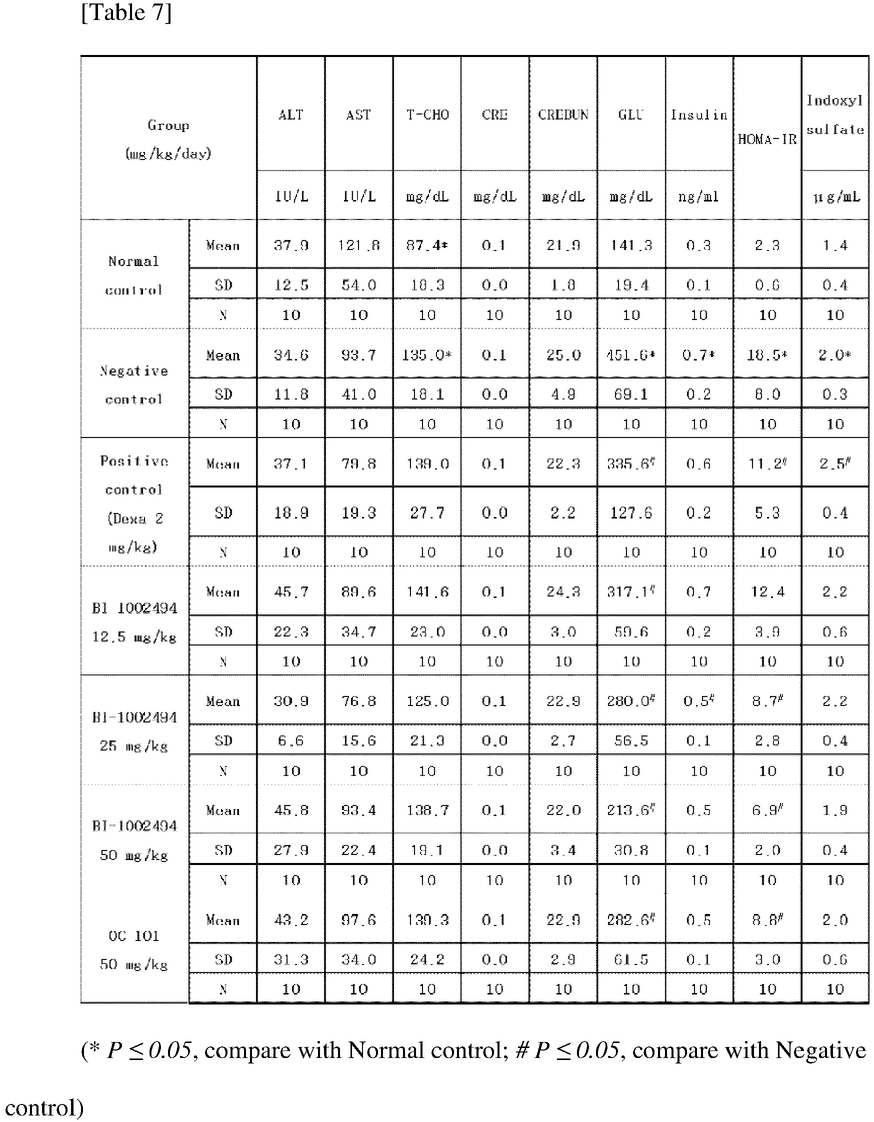

As a result, in the negative control group, T-CHO, GLU, insulin, HOMA-IR, and Indoxyl sulfate were increased statistically significantly compared to those of the normal control group, and in the positive control group, GLU and HOMA-IR were decreased statistically significantly and Indoxyl sulfate was increased statistically significantly compared to those of the negative control group (Table 7). In addition, in all administered groups of (R)-4-{(R)-1-[7-(3,4,5-trimethoxy-phenyl)-[1,6] naphthyridin-5-yloxy]-ethyl}pyrrolidin-2-one, GLU was statistically significantly reduced, and a clear dose dependence was observed. In addition, in 25 and 50 mg/kg administered groups of (R)-4-{(R)-1-[7-(3,4,5-trimethoxy-phenyl)-[1,6] naphthyridin-5-yloxy]-ethyl}pyrrolidin-2-one, HOMA-IR was statistically significantly reduced as compared with that of the negative control group (Table 7). In addition, in the OC-101 administered group, GLU and HOMA-IR were decreased statistically significantly compared to those of the negative control group (Table 7).

### Example 5. Confirmation of histopathological change

At the end of the observation period, the mice in each group of Example 1 were confirmed to be dead, and the liver, kidney, and pancreas were extracted and fixed with 10% neutral buffered formalin (NBF) in at least 20 times the tissue volume. The fixed tissue was sectioned to a thickness of approximately 4 mm and then produced into a paraffin block through general tissue processing and embedding processes. Thereafter, the tissue was sectioned to a thickness of 4 µm using a rotary microtome, and then H&E staining was performed, and histopathological changes were observed.

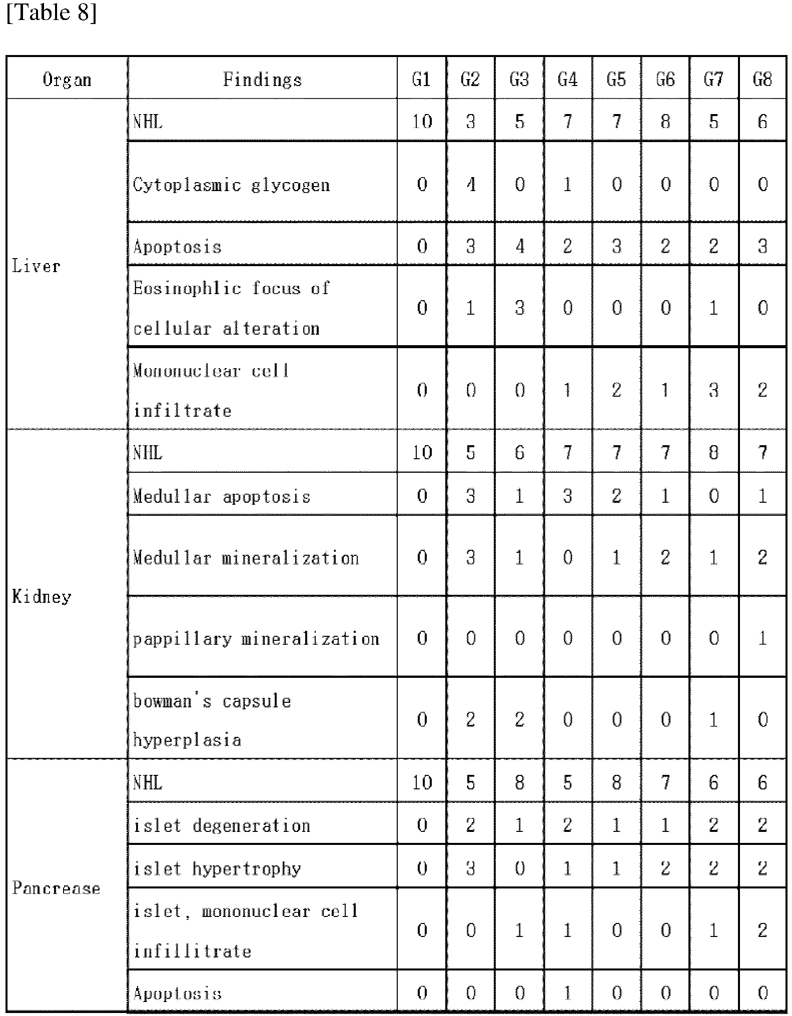

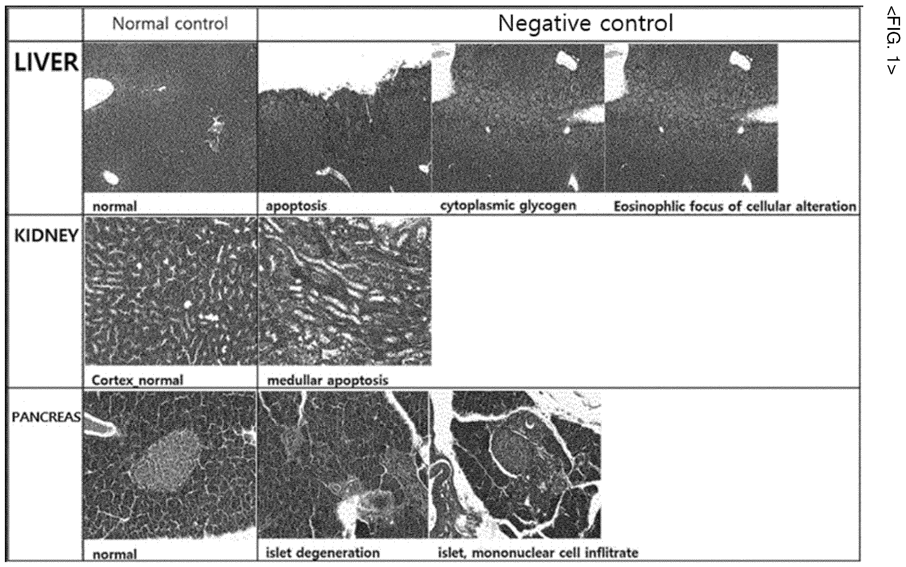

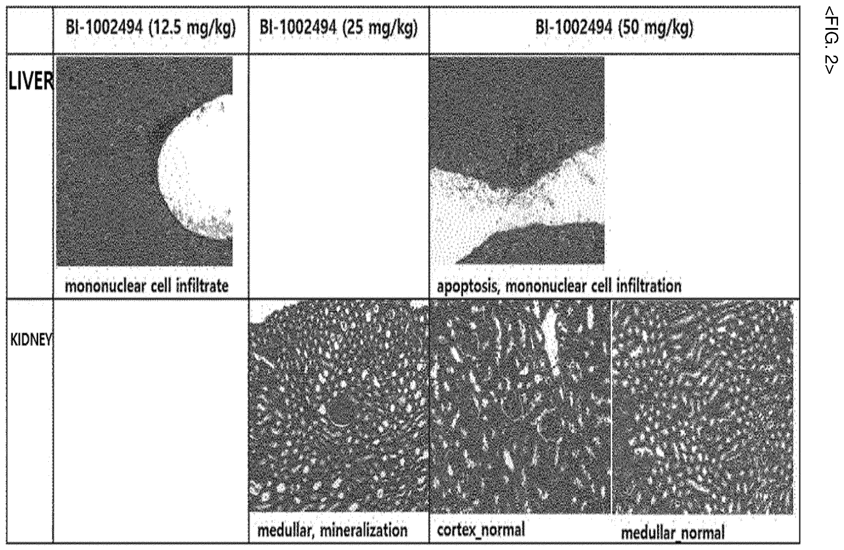

As a result, in the negative control group, compared to the normal control group, cytoplasmic glycogen, apoptosis, and the like were observed in the liver, medullar apoptosis and medullar mineralization were observed in the kidney, and islet degeneration and islet hypertrophy were observed in the pancreas (Table 8 and FIG. 1). Further, in the administered group of (R)-4-{(R)-1-[7-(3,4,5-trimethoxy-phenyl)-[1,6] naphthyridin-5-yloxy]-ethyl}pyrrolidin-2-one, the cytoplasmic glycogen was reduced in the liver and the medullar apoptosis was reduced in the kidney in a dose-dependent manner (Table 8 and FIG. 2).

Through the result, it was confirmed that in disease induction using STZ, in the negative control group, compared to the normal control group, plasma glucose and insulin were increased, insulin resistance (HOMA-IP(IR)) was increased, the apoptosis of the kidney was confirmed, glomerular basement membrane hypertrophy was observed, and the features of diabetic renal diseases were exhibited. In addition, when administering (R)-4-{(R)-1-[7-(3,4,5-trimethoxy-phenyl)-[1,6] naphthyridin-5-yloxy]-ethyl}pyrrolidin-2-one after such induction of diabetic renal diseases, it can be seen that blood glucose levels, and glucose and insulin resistance (HOMA-IR) were decreased in a dose-dependent manner, and (R)-4-{(R)-1-[7-(3,4,5-trimethoxyphenyl)-[1,6] naphthyridin-5-yloxy]-ethyl}pyrrolidin-2-one is effective in treatment of diabetic renal diseases.

## Claims

1. A pharmaceutical composition for preventing or treating kidney diseases comprising (R)-4-{(R)-1-[7-(3,4,5-trimethoxy-phenyl)-[1,6] naphthyridin-5-yloxy]-ethyl}pyrrolidin-2-one or a pharmaceutically acceptable salt thereof as an active ingredient.

2. The pharmaceutical composition for preventing or treating kidney diseases of claim 1, wherein the (R)-4-{(R)-1-[7-(3,4,5-trimethoxy-phenyl)-[1,6] naphthyridin-5-yloxy]-ethyl}pyrrolidin-2-one is a compound represented by Chemical Formula 1 below:

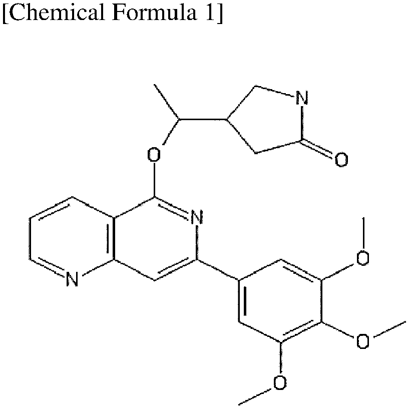

3. The pharmaceutical composition for preventing or treating kidney diseases of claim 1, wherein the kidney diseases are one or more selected from the group consisting of acute kidney injury, end-stage kidney disease (ESKD), renal failure, systemic lupus erythematosus, diabetic renal disease (DN), IgA nephritis (IgAN), HIV-related nephropathy, chronic kidney disease, focal segmental glomerulosclerosis (FSGS), minimal change nephrotic syndrome (MCD), and xanthine oxidase deficiency.

4. The pharmaceutical composition for preventing or treating kidney diseases of claim 1, wherein the kidney disease is Type 2 diabetic renal disease (diabetic nephropathy).

5. The pharmaceutical composition for preventing or treating kidney diseases of claim 1, wherein the composition inhibits apoptosis of the renal medulla.

6. The pharmaceutical composition for preventing or treating kidney diseases of claim 1, wherein the composition inhibits calcification of the renal medulla.

7. The pharmaceutical composition for preventing or treating kidney diseases of claim 1, further comprising: cromolyn.

8. A food composition for preventing or palliating kidney diseases comprising (R)-4-{(R)-1-[7-(3,4,5-trimethoxy-phenyl)-[1,6] naphthyridin-5-yloxy]-ethyl}pyrrolidin-2-one or a pharmaceutically acceptable salt thereof as an active ingredient.

9. The food composition for preventing or palliating kidney diseases of claim 8, wherein the kidney disease is diabetic renal disease (diabetic nephropathy).

10. The food composition for preventing or palliating kidney diseases of claim 8, further comprising: cromolyn.

11. A method for treating kidney diseases comprising administering (R)-4-{(R)-1-[7-(3,4,5-trimethoxy-phenyl)-[1,6] naphthyridin-5-yloxy]-ethyl}pyrrolidin-2-one or a pharmaceutically acceptable salt thereof in a pharmaceutically effective dose to a subject with kidney diseases.

12. The method for treating kidney diseases of claim 11, further comprising: administering cromolyn or a pharmaceutically acceptable salt thereof in a pharmaceutically effective dose to a subject with kidney diseases.

13. A use of (R)-4-{(R)-1-[7-(3,4,5-trimethoxy-phenyl)-[1,6] naphthyridin-5-yloxy]-ethyl}pyrrolidin-2-one or a pharmaceutically acceptable salt thereof for use in the preparation of a pharmaceutical composition for the prevention and treatment of kidney diseases.

14. A use of (R)-4-{(R)-1-[7-(3,4,5-trimethoxy-phenyl)-[1,6] naphthyridin-5-yloxy]-ethyl}pyrrolidin-2-one and cromolyn or pharmaceutically acceptable salts thereof for use in the preparation of a pharmaceutical composition for the prevention and treatment of kidney diseases.
